# EUROPÄISCHE PATENTSCHRIFT

(11) **EP 1 589 907 B1**
(45) Veröffentlichungstag und Bekanntmachung des Hinweises auf die Patenterteilung: **28.03.2012**
(21) Anmeldenummer: 04700261.3
(22) Anmeldetag: 16.01.2004
(51) Int. Cl.: A61F 2/38

(54) **IMPLANTAT**
IMPLANT
IMPLANT

(30) Priorität: 05.02.2003 DE 10305591
(43) Veröffentlichungstag der Anmeldung: 02.11.2005
(73) Patentinhaber: Aesculap AG, 78532 Tuttlingen (DE)
(72) Erfinder: REICH, Jan, 78073 Hochemmingen (DE); HAGEN, Thomas, 78532 Tuttlingen (DE)
(74) Vertreter: Pioch, Holger
(86) Internationale Anmeldenummer: PCT/EP2004/000274
(87) Internationale Veröffentlichungsnummer: WO 2004/069103

(56) Entgegenhaltungen:
- EP-A- 0 709 075
- FR-A- 2 554 709
- US-A- 4 888 020
- US-A- 5 226 916

## Beschreibung

Die Erfindung betrifft ein Implantat wie im Anspruch 1 definiert zum Ersetzen mindestens eines Teils einer natürlichen Gelenkfläche eines einen Teil eines Gelenk bildenden Knochens umfassend eine am verbliebenen Knochen anliegende Verankerungsfläche und eine vom Knochen weg weisende künstliche Gelenkoberfläche, wobei die Verankerungsfläche mindestens einen zylindrisch geformten Bereich umfaßt, der eine Zylinderachse definiert.

Derartige Implantate werden beispielsweise bei künstlichen Kniegelenken verwendet. Mit diesen Implantaten lassen sich die infolge einer übermäßigen Abnützung geschädigte Gelenkoberflächen des Femurs, also eine oder beide Kondylen des Femurs, ersetzen. Sie sind beispielsweise aus der US 5,226,916 bekannt.

Verankerungsflächen von Implantaten zum Ersetzen mindestens eines Teils einer natürlichen Gelenkfläche eines einen Teil eines Gelenk bildenden Knochens umfassend eine am verbliebenen Knochen anliegende Verankerungsfläche und eine vom Knochen weg weisende künstliche Gelenkoberfläche sind wie in der EP 0 327 387 B1 beschrieben, sphärisch geformt oder aus mehreren, relativ zueinander geneigten ebenen Flächen gebildet. Weitere Kniegelenkendoprothesen sind in der FR 2 554 709 A1, der EP 0 709 075 A1 sowie der US 4,888,020 beschrieben.

Als nachteilig bei Implantaten mit mehreren ebenen Gelenkflächen hat sich erwiesen, daß mehr Knochensubstanz als eigentlich erforderlich vor dem Einsetzen des Implantats entfernt werden muß. Dies ist zwar bei Implantaten mit sphärisch geformten Verankerungsflächen nicht der Fall, allerdings muß zur Bearbeitung des Knochens ein sphärischer Fräskopf von ventral eingeführt werden. Dies macht die nahezu vollständige Eröffnung des Kniegelenks erforderlich, um den Femurknochen für das Einsetzen des Implantats vorzubereiten.

Es ist daher Aufgabe der vorliegenden Erfindung, ein Implantat der eingangs beschriebenen Art so zu verbessern, daß möglichst wenig Knochensubstanz auf einfache Weise zu entfernen ist.

Diese Aufgabe wird bei einem gattungsgemäßen Implantat erfindungsgemäß dadurch gelöst, daß die Gelenkoberfläche von der Zylinderachse weg weisend konvex gekrümmt ist, daß die künstliche Gelenkoberfläche eine sphärische Form aufweist und daß die Zylinderachse den Mittelpunkt der sphärisch geformten, künstlichen Gelenkoberfläche enthält.

Dadurch, daß das Implantat einen zylindrisch geformten Bereich umfaßt, muß am Knochen nur eine zur Verankerungsfläche korrespondierende zylindrische Oberfläche vorbereitet werden, bei der weniger Knochensubstanz entfernt werden muß, als für das Einsetzen von Implantaten mit mehreren ebenen Verankerungsflächen erforderlich wäre. Ferner hat eine zylindrische Oberfläche den Vorteil, daß diese nicht von ventral, sondern von lateral oder medial bearbeitet werden kann. Dies macht es nicht mehr unbedingt erforderlich, das gesamte Knie zu eröffnen, vielmehr ist auf diese Weise ein minimalinvasiver Eingriff am Kniegelenk möglich. Vorteilhaft ist es, daß die künstliche Gelenkoberfläche von der Zylinderachse weg weisend konvex gekrümmt ist. Durch diese Ausgestaltung wird die künstliche Gelenkoberfläche der natürlichen Form sehr nahekommend ausgebildet. Günstig ist es, daß die künstliche Gelenkoberfläche eine sphärische Form auf. Eine sphärische Form läßt sich besonders einfach herstellen und kann auf einfache Weise von einem kugelig ausgenommenen, als künstlicher Meniskus dienendes Gleitelement im Wesentlichen vollständig während einer Abwinkelbewegung des Knies geführt werden. Ein besonders einfacher Aufbau des Implantats ergibt sich dadurch, daß die Zylinderachse den Mittelpunkt der sphärisch geformten, künstlichen Gelenkoberfläche enthält. Außerdem ergibt sich so eine schalenförmige Ausgestaltung des Implantats, die eine minimale Resektion von Knochensubstanz erforderlich macht.

Günstig ist es, wenn die gesamte Verankerungsfläche zylindrisch geformt ist. Dadurch wird der Aufbau des Implantats und damit auch die Herstellung desselben vereinfacht. Außerdem muß zum Einsetzen eines solchen Implantats am wenigsten Knochensubstanz reseziert werden.

Gemäß einer bevorzugten Ausführungsform der Erfindung kann vorgesehen sein, daß die Verankerungsfläche einen planen Bereich umfaßt und daß sich der plane Bereich an den zylindrisch geformten Bereich anschließt. Durch das Vorsehen des planen Bereichs der Verankerungsfläche lassen sich Hinterschneidungen am zu resezierenden Knochen vermeiden.

Günstig ist es, wenn im Übergangsbereich zwischen dem planen Bereich und dem zylindrisch geformten Bereich der plane Bereich eine Tangentialebene an den zylindrisch geformten Bereich bildet. Dadurch läßt sich die Verankerungsfläche völlig knick- und kantenfrei ausbilden. Wird dagegen im Übergangsbereich zwischen dem planen Bereich und dem zylindrisch geformten Bereich der plane Bereich unter einem Winkel an eine Tangentialebene an den zylindrisch geformten Bereich angeformt, so paßt sich das Implantat an die verbliebene Knochenoberfläche besonders gut an, wenn die plane Fläche um einige Grad nach dorsal verkippt ist, beispielsweise um 10°. Auf diese Weise muß Knochensubstanz nur in minimalem Umfang entfernt werden. Zusätzlich werden Hinterschneidungen vermieden.

Gemäß einer bevorzugten Ausführungsform der Erfindung kann vorgesehen sein, daß das Implantat einen planen Verankerungsabschnitt und einen gekrümmten Verankerungsabschnitt aufweist, daß der plane Verankerungsabschnitt den planen Bereich und daß der gekrümmte Verankerungsabschnitt den zylindrisch geformten Bereich umfaßt. Dadurch ergibt sich ein besonders einfacher Aufbau des Implantats.

Noch einfacher wird der Aufbau des Implantats, wenn der plane Verankerungsabschnitt eine Symmetrieebene aufweist, die quer zur Zylinderachse verläuft. Der gekrümmte Verankerungsabschnitt kann dabei beliebig geformt sein.

Besonders einfach in der Herstellung und im Aufbau wird das Implantat, wenn der gekrümmte Verankerungsabschnitt eine Symmetrieebene aufweist, die quer zur Zylinderachse verläuft.

Vorzugsweise weist das Implantat eine Symmetrieebene auf, die quer zur Zylinderachse verläuft. Damit ergibt sich insgesamt sowohl konstruktiv als auch herstellungstechnisch ein besonders einfacher Aufbau des Implantats.

Günstig ist es, wenn das Implantat Seitenflächen aufweist, welche die Gelenkoberfläche und die Verankerungsfläche miteinander verbinden. Dies ermöglicht es, die Ausbildung von Kanten im Übergangsbereich zwischen der Gelenkoberfläche und der Verankerungsfläche zu vermeiden oder Anschlagflächen in Form der Seitenflächen auszubilden.

Besonders wenig Knochensubstanz muß reseziert werden, wenn die Seitenflächen parallel zur Richtung der Zylinderachse einen konstanten Abstand voneinander aufweisen.

Gemäß einer weiteren bevorzugten Ausführungsform der Erfindung kann vorgesehen sein, daß die Seitenflächen mindestens abschnittsweise gekrümmt sind, so daß sich ein Abstand der Seitenflächen zu einer quer zur Zylinderachse verlaufenden Ebene ändert. Damit ergibt sich ein Aufbau des Implantats, der je nach zu ersetzender natürlicher Kondyle minimale Knochensubstanz zu resezieren erfordert. Ferner lassen sich so bei einem Großteil der Patienten Kondylenflächen am Natürlichsten wieder herstellen.

Eine einer natürlichen Kondylenform besonders ähnliche Form des Implantats ergibt sich, wenn der Abstand der Seitenflächen ausgehend vom planen Verankerungsabschnitt zu der quer zur Zylinderachse verlaufenden Ebene zunimmt.

Grundsätzlich könnten die Seitenflächen des gekrümmten Verankerungsabschnitts bezogen auf die quer zur Zylinderachse verlaufende Ebene parallel ausgerichtet sein. Günstig ist es, wenn die Seitenflächen des gekrümmten Verankerungsabschnitts bezogen auf die quer zur Zylinderachse verlaufende Ebene. Dadurch läßt sich eine natürliche Kondylenform weitgehend rekonstruieren.

Ein besonders einfacher Aufbau des Implantats ergibt sich, wenn eine Krümmung der Seitenflächen konstant ist.

Vorteilhaft ist es, wenn der Abstand zwischen der Gelenkoberfläche und dem zylindrisch geformten Bereich der Verankerungsfläche in einer quer zur Zylinderachse verlaufenden Ebene konstant ist. Dadurch wird nur eine minimale Resektion des Knochens vor dem Einsetzen des Implantats erforderlich.

Günstig ist es, wenn der Abstand zwischen der Gelenkoberfläche und dem zylindrisch geformten Bereich der Verankerungsfläche in einer quer zur Zylinderachse verlaufenden Symmetrieebene des Implantats am größten ist. Auf diese Weise läßt sich die Stabilität des Implantats in vorteilhafter Weise erhöhen. Durch die symmetrische Ausbildung des Implantats wird zudem die Menge des zu entfernenden Knochens minimiert.

Besonders von Vorteil ist es, wenn sich der zylindrisch geformte Bereich der Verankerungsfläche maximal über einen Winkelbereich von 180° erstreckt. Dadurch werden Hinterschneidungen des Implantats an dem einen Teil des Gelenks bildenden Knochens vermieden. Vorzugsweise erstreckt sich der Winkelbereich über maximal 120°.

Ein besonders guter Halt des Implantats am Femur ergibt sich, wenn der Winkelbereich etwa 110° umfaßt.

Gemäß einer bevorzugten Ausführungsform der Erfindung kann vorgesehen sein, daß das Implantat ein unikondyläres Implantat zum Ersetzen einer Kondylenoberfläche eines Femurknochens ist. Diese Ausgestaltung des Implantats ermöglicht es auf einfache Weise, nur einen Teil des Femurknochens, also nur eine der zwei vorhandenen Kondylen zu ersetzen, falls dies aufgrund einer einseitigen übermäßigen Abnutzung der natürlichen Gelenkoberfläche ausreichend ist.

Um bei einer Operation, bei der ein Gelenk mindestens teilweise ersetzt werden soll, unabhängig von der Größe des Gelenks stets das optimale Implantat einsetzen zu können, ist es vorteilhaft, wenn ein Satz von Implantaten vorgesehen ist, wobei Krümmungsradien der Gelenkoberflächen jeweils um 5 mm variieren, insbesondere um 3 mm, vorzugsweise um 1 mm.

Um je nach Bedarf sowohl laterale als auch mediale Kondylenflächen einzeln zu ersetzen, ist es von Vorteil, wenn der Implantatsatz zueinander symmetrisch ausgebildete Implantate umfaßt.

Die nachfolgende Beschreibung einer bevorzugten Ausführungsform der Erfindung dient im Zusammenhang mit der Zeichnung der näheren Erläuterung. Es zeigen:
- Figur 1:: eine perspektivische Ansicht des Implantats;
- Figur 2:: eine weitere perspektivische Ansicht des Implantats aus Figur 1;
- Figur 3:: eine perspektivische Ansicht eines teilweise resezierten distalen Endes eines Femurknochens;
- Figur 4:: eine Seitenansicht des Femurknochens aus Figur 3 mit einem daran angeordneten Implantat;
- Figur 5:: eine Draufsicht auf eine ebene Verankerungsfläche eines zweiten Ausführungsbeispiels eines Implantats;
- Figur 6:: eine Ansicht des Implantats aus Figur 5 in Richtung des Pfeils A;
- Figur 7:: eine Ansicht eines Femurknochens von vorn mit einem daran angeordneten, in den Figuren 5 und 6 dargestellten Implantat; und
- Figur 8:: eine Ansicht ähnlich Figur 7 mit einem dritten Ausführungsbeispiel eines Implantats.

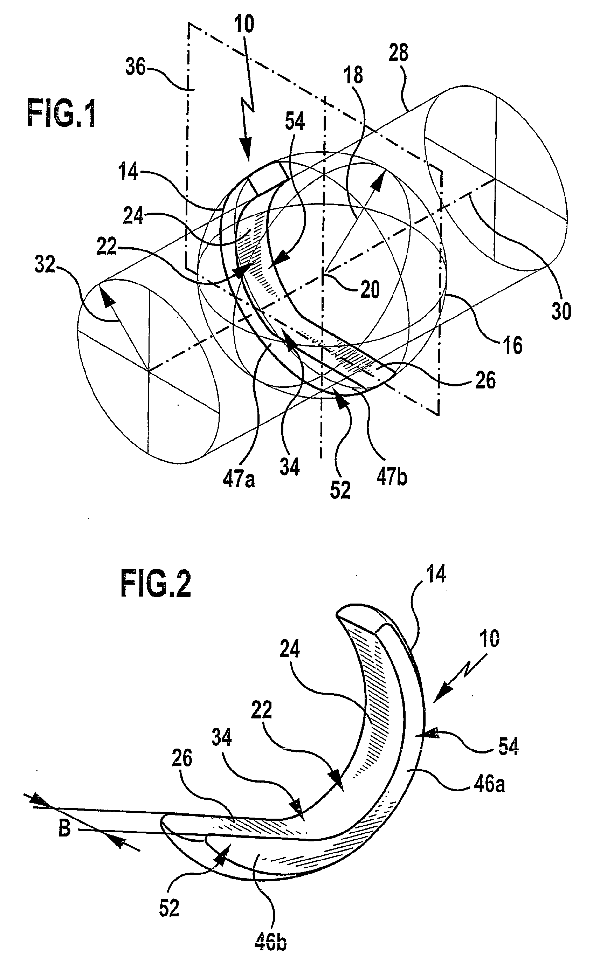

In den Figuren 1 und 2 ist ein insgesamt mit dem Bezugszeichen 10 versehenes unikondyläres Implantat zum Ersetzen mindestens eines Teils einer natürlichen Kondylenfläche eines einen Teil eines Kniegelenk bildenden Femurknochens 12 dargestellt. Das Implantat 10 weist eine konvex gekrümmte künstliche Gelenkoberfläche 14 auf, die auch als Artikulationsfläche bezeichnet wird. Die Gelenkoberfläche 14 bildet einen Ausschnitt einer Sphäre 16 mit einem Sphärenradius 18 und einem Sphärenmittelpunkt 20.

Eine insgesamt mit dem Bezugszeichen 22 versehene Verankerungsfläche weist einen konkaven Bereich 24 und einen planen Bereich 26 auf. Der konkave Bereich ist Teil eines Zylinders 28, dessen Symmetrieachse, also die Zylinderachse 30, den Sphärenmittelpunkt 20 enthält. Der konkave Bereich 24 erstreckt sich über einen Winkelbereich 25, der kleiner ist als 180°, vorzugsweise etwa 110° beträgt, wie beim in den Figuren 1, 2 und 4 dargestellten Ausführungsbeispiel. Der Zylinderradius 32 ist etwas kleiner als der Sphärenradius 18. Der konkave Bereich 24 geht in einem Übergangsbereich 34 tangential in den planen Bereich 26 über, und zwar ohne Ausbildung einer Kante.

Ferner weist das Implantat 10 Seitenflächen 46a und 46b sowie 47a und 47b auf, wobei die Seitenflächen 46a und 47a die Gelenkoberfläche 14 im Bereich eines gekrümmten Verankerungsabschnitts 54 des Implantats 10 mit dem konkaven Bereich 24 verbinden, die Seitenflächen 46b und 47b die Gelenkoberfläche 14 mit dem planen Bereich 26 im Bereich eines planen Verankerungsabschnitts 52 des Implantats 10. Dabei ist der Abstand B der Seitenflächen 46a und 47a voneinander sowie der Abstand B der Seitenflächen 46b und 47b voneinander im wesentlichen konstant. Ferner verlaufen die Seitenflächen 46 und 47 im wesentlichen parallel zueinander.

Das Implantat 10 ist insgesamt spiegelsymmetrisch zu einer Spiegelebene 36 ausgebildet, die senkrecht zur Zylinderachse 30 orientiert ist und den Sphärenmittelpunkt 20 enthält.

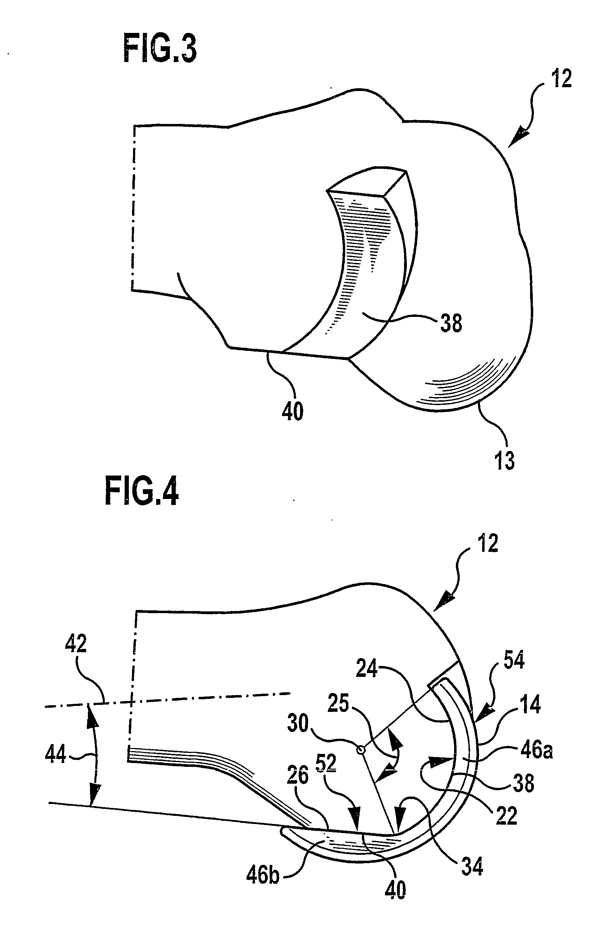

Um das Implantat 10 mit dem Femurknochen 12 zu verbinden, wird eine der beiden ursprünglich vorhandenen Kondylen 13 teilweise reseziert, und zwar derart, daß eine konvex geformte, zylindrische Anlagefläche 38 am Knochen verbleibt. An diese, in Figur 3 dargestellte Anlagefläche 38 schließt sich ein ebener Abschnitt 40 am Knochen an, der so ausgebildet ist, daß er im wesentlichen zum planen Bereich 26 des Implantats 10 korrespondiert. Die Anlagefläche 38 und insbesondere der ebene Abschnitt 40 sind so angeordnet, daß eine vom ebenen Abschnitt 40 definierte Ebene relativ zu einer Femurlängsachse 42 um einen Winkel 44 nach dorsal verkippt ist, der idealerweise etwa 10° beträgt. Dadurch werden Hinterschneidungen am Knochen vermieden.

Der größte Abstand zwischen der Verankerungsfläche 22 und der Gelenkoberfläche 14 besteht in der Symmetrieebene 36, insbesondere zwischen dem planen Bereich 26 und der Gelenkoberfläche 14.

Die Anlagefläche 38 kann beispielsweise mit einem seitlich angesetzten Fräswerkzeug präpariert werden. Eine Eröffnung des Kniegelenks von ventral ist dabei nicht erforderlich. Ferner kann das Implantat 10 auch von lateral bzw. medial an die Anlagefläche 38 und den ebenen Abschnitt 40 des Femurknochens 12 herangeführt und bei Bedarf zementiert werden.

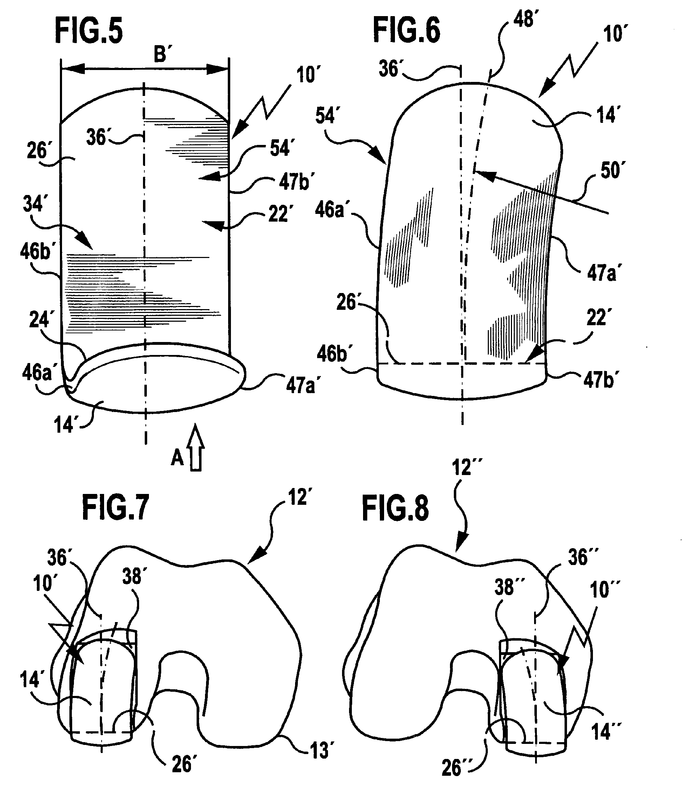

In den Figuren 5 bis 7 ist ein zweites Ausführungsbeispiel eines erfindungsgemäßen Implantats dargestellt und insgesamt mit dem Bezugszeichen 10' versehen. Es entspricht im wesentlichen dem im Zusammenhang mit den Figuren 1 bis 4 beschriebenen Implantat 10, so daß gleiche Teile der Implantate 10 und 10' mit denselben Bezugszeichen versehen sind.

Anders als das Implantat 10 ist das Implantat 10' nicht vollständig symmetrisch bezüglich einer Symmetrieebene 36' ausgebildet. Lediglich der Teil des Implantats 10', welcher den planen Bereich 26' umfaßt, also der plane Verankerungsabschnitt 52', ist symmetrisch zur Ebene 36' ausgebildet. Dagegen ist der gekrümmte Verankerungsabschnitt 54' umfassend den konkaven Bereich 24' bezogen auf die Symmetrieebene 36' gekrümmt und zwar so, daß ein Schwerpunkt des gekrümmten Verankerungsbereichs 54' nicht auf, sondern neben der Symmetrieebene 36' liegt. Seitenflächen 46a' und 47a' bzw. 46b' und 47b', die die konvex gekrümmte künstliche Gelenkoberfläche 14' mit dem konkaven Bereich 24' und dem planen Bereich 26' verbinden, weisen voneinander einen konstanten Abstand B' auf, so daß das Implantat 10', wie auch das Implantat 10, eine quer zur Symmetrieebene 36' konstante Breite aufweist.

Die Artikulationsfläche 14' ist sphärisch geformt, so daß bei ebenfalls zylindrisch geformtem konkaven Bereich 24' die Breite der Seitenfläche 46a' quer zur Artikulationsfläche 14' größer ist als die Breite der Seitenfläche 47a'. Wie in Figur 6 dargestellt, ist eine Mittellinie 48' des Implantats 10' von der Symmetrieebene 36' weg gekrümmt. Der Krümmungsradius 50' der Mittellinie 48' beträgt etwa 150 mm und ist konstant, kann aber auch mit zunehmendem Abstand von dem planen Bereich 26' abnehmen oder zunehmen.

Das Implantat 10' ersetzt wie in Figur 7 dargestellt, eine teilweise resezierte natürliche Kondyle 13' des Femurs 12', wobei aufgrund des gekrümmten Verankerungsbereichs 54' eine bessere Anpassung an die natürlichen Verhältnisse erreicht wird als beim Implantat 10.

Wegen des insgesamt unsymmetrischen Aufbaus des Implantats 10' werden für eine optimale Rekonstruktion beider natürlicher Kondylenflächen eines Femurs zwei unterschiedlich gekrümmte Implantate benötigt. Ein Beispiel für ein zum Implantat 10' insgesamt spiegelsymmetrisch aufgebautes Implantat 10" ist in Figur 8 dargestellt. Damit läßt sich eine Kondylenfläche entsprechend der natürlichen Anatomie ersetzen.

## Patentansprüche

1. Implantat (10) zum Ersetzen mindestens eines Teils einer natürlichen Gelenkfläche eines einen Teil eines Gelenk bildenden Knochens (12) umfassend eine am verbliebenen Knochen (12) anliegende Verankerungsfläche (22) und eine vom Knochen (12) weg weisende künstliche Gelenkoberfläche (14), wobei die Verankerungsfläche (22) mindestens einen zylindrisch geformten Bereich (24) umfaßt, der eine Zylinderachse (30) definiert, wobei die künstliche Gelenkoberfläche (14) von der Zylinderachse (30) weg weisend konvex gekrümmt ist, und die künstliche Gelenkoberfläche (14) eine sphärische Form aufweist und die Zylinderachse (30) den Mittelpunkt (20) der sphärisch geformten, künstlichen Gelenkoberfläche (14) enthält.

2. Implantat nach Anspruch 1, **dadurch gekennzeichnet, daß** die gesamte Verankerungsfläche (22) zylindrisch geformt ist.

3. Implantat nach Anspruch 1 **dadurch gekennzeichnet, daß** die Verankerungsfläche (22) einen planen Bereich (26) umfaßt und daß sich der plane Bereich (26) an den zylindrisch geformten Bereich (24) anschließt.

4. Implantat nach Anspruch 3, **dadurch gekennzeichnet, daß** im Übergangsbereich (34) zwischen dem planen Bereich (26) und dem zylindrisch geformten Bereich (24) der plane Bereich (26) eine Tangentialebene an den zylindrisch geformten Bereich (24) bildet.

5. Implantat nach einem der Ansprüche 3 oder 4, **dadurch gekennzeichnet, daß** das Implantat (10) einen planen Verankerungsabschnitt (52) und einen gekrümmten Verankerungsabschnitt (54) aufweist, daß der plane Verankerungsabschnitt (52) den planen Bereich (26) und daß der gekrümmte Verankerungsabschnitt (54) den zylindrisch geformten Bereich (24) umfaßt.

6. Implantat nach Anspruch 5, **dadurch gekennzeichnet, daß** der plane Verankerungsabschnitt (52) eine Symmetrieebene (36) aufweist, die quer zur Zylinderachse (30) verläuft.

7. Implantat nach einem der Ansprüche 6 oder 7, **dadurch gekennzeichnet, daß** der gekrümmte Verankerungsabschnitt (54) eine Symmetrieebene (36) aufweist, die quer zur Zylinderachse verläuft.

8. Implantat nach einem der voranstehenden Ansprüche, **dadurch gekennzeichnet, daß** das Implantat (10) eine Symmetrieebene (36) aufweist, die quer zur Zylinderachse verläuft.

9. Implantat nach einem der voranstehenden Ansprüche, **dadurch gekennzeichnet, daß** das Implantat (10) Seitenflächen (46a, 46b, 47a, 47b) aufweist, welche die künstliche Gelenkoberfläche (14) und die Verankerungsfläche (22) miteinander verbinden.

10. Implantat nach Anspruch 9, **dadurch gekennzeichnet, daß** die Seitenflächen (46a, 46b, 47a, 47b) parallel zur Richtung der Zylinderachse (30) einen konstanten Abstand (B) voneinander aufweisen.

11. Implantat nach einem der Ansprüche 9 oder 10, **dadurch gekennzeichnet, daß** die Seitenflächen (46a, 47a) mindestens abschnittsweise gekrümmt sind, so daß sich ein Abstand der Seitenflächen (46a, 47a) zu einer quer zur Zylinderachse (30) verlaufenden Ebene ändert.

12. Implantat nach Anspruch 11, **dadurch gekennzeichnet, daß** der Abstand der Seitenflächen (46a, 47a) ausgehend vom planen Verankerungsabschnitt (52) zu der quer zur Zylinderachse (30) verlaufenden Ebene zunimmt.

13. Implantat nach einem der Ansprüche 11 oder 12, **dadurch gekennzeichnet, daß** die Seitenflächen (46a, 47a) des gekrümmten Verankerungsabschnitts (54) bezogen auf die quer zur Zylinderachse (30) verlaufende Ebene (36) gekrümmt sind.

14. Implantat nach Anspruch 12, **dadurch gekennzeichnet, daß** eine Krümmung der Seitenflächen (46a, 47a) konstant ist.

15. Implantat nach einem der voranstehenden Ansprüche, **dadurch gekennzeichnet, daß** der Abstand zwischen der künstlichen Gelenkoberfläche (14) und dem zylindrisch geformten Bereich (24) der Verankerungsfläche (22) in einer quer zur Zylinderachse (30) verlaufenden Ebene konstant ist.

16. Implantat nach einem der voranstehenden Ansprüche, **dadurch gekennzeichnet, daß** der Abstand zwischen der künstlichen Gelenkoberfläche (14) und dem zylindrisch geformten Bereich (24) der Verankerungsfläche (22) in einer quer zur Zylinderachse (30) verlaufenden Symmetrieebene (36) des Implantats (10) am größten ist.

17. Implantat nach einem der voranstehenden Ansprüche, **dadurch gekennzeichnet, daß** sich der zylindrisch geformte Bereich (24) der Verankerungsfläche (22) maximal über einen Winkelbereich (25) von 180° erstreckt, insbesondere über ein Winkelbereich von maximal 120°.

18. Implantat nach Anspruch 17, **dadurch gekennzeichnet, daß** der Winkelbereich (25) etwa 110° umfaßt.

19. Implantat nach einem der voranstehenden Ansprüche, **dadurch gekennzeichnet, daß** das Implantat ein unikondyläres Implantat (10) zum Ersetzen einer Kondylenoberfläche (13) eines Femurknochens (12) ist.

20. Implantatsatz umfassend eine Mehrzahl von Implantaten nach einem der voransstehenden Ansprüche, wobei Krümmungsradien der künstlichen Gelenkoberflächen (14) im Abstand von 5 mm, insbesondere um 3 mm, vorzugsweise um 1 mm variieren.

21. Implantatsatz nach Anspruch 20, **dadurch gekennzeichnet, daß** der Implantatsatz zueinander symmetrisch ausgebildete Implantate (10', 10") umfaßt.

## Claims

1. Implant (10) for the replacement of at least a part of a natural articular surface of a bone (12) forming a part of a joint comprising an anchorage surface (22) abutting against the remaining bone (12) and an artificial articular surface (14) directed away from the bone (12), wherein the anchorage surface (22) comprises at least one cylindrically shaped area (24), which defines a cylinder axis (30), wherein the artificial articular surface (14) is convexly curved directed away from the cylinder axis (30), and the artificial articular surface (14) has a spherical shape, and the cylinder axis (30) contains the centre point (20) of the spherically shaped, artificial articular surface (14).

2. Implant according to claim 1, **characterized in that** the entire anchorage surface (22) is cylindrically shaped.

3. Implant according to claim 1, **characterized in that** the anchorage surface (22) comprises a plane area (26), and **in that** the plane area (26) adjoins the cylindrically shaped area (24).

4. Implant according to claim 3, **characterized in that** in the transition area (34) between the plane area (26) and the cylindrically shaped area (24) the plane area (26) forms a tangential plane onto the cylindrically shaped area (24).

5. Implant according to claim 3 or 4, **characterized in that** the implant (10) has a plane anchorage section (52) and a curved anchorage section (54), **in that** the plane anchorage section (52) comprises the plane area (26), and **in that** the curved anchorage section (54) comprises the cylindrically shaped area (24).

6. Implant according to claim 5, **characterized in that** the plane anchorage section (52) has a plane of symmetry (36), which extends transversely to the cylinder axis (30).

7. Implant according to claim 6 or 7, **characterized in that** the curved anchorage section (54) has a plane of symmetry (36), which extends transversely to the cylinder axis.

8. Implant according to any one of the preceding claims, **characterized in that** the implant (10) has a plane of symmetry (36), which extends transversely to the cylinder axis.

9. Implant according to any one of the preceding claims, **characterized in that** the implant (10) has side faces (46a, 46b, 47a, 47b), which connect the artificial articular surface (14) and the anchorage surface (22) to one another.

10. Implant according to claim 9, **characterized in that** the side faces (46a, 46b, 47a, 47b) are at a constant spacing (B) from one another parallel to the direction of the cylinder axis (30).

11. Implant according to claim 9 or 10, **characterized in that** the side faces (46a, 47a) are curved at least in sections, so that a spacing of the side faces (46a, 47a) from a plane extending transversely to the cylinder axis (30) varies.

12. Implant according to claim 11, **characterized in that**, starting from the plane anchorage section (52), the spacing of the side faces (46a, 47a) increases in a direction towards the plane extending transversely to the cylinder axis (30).

13. Implant according to claim 11 or 12, **characterized in that** the side faces (46a, 47a) of the curved anchorage section (54) are curved in relation to the plane (36) extending transversely to the cylinder axis (30).

14. Implant according to claim 12, **characterized in that** a curvature of the side faces (46a, 47a) is constant.

15. Implant according to any one of the preceding claims, **characterized in that** the spacing between the artificial articular surface (14) and the cylindrically shaped area (24) of the anchorage surface (22) is constant in a plane extending transversely to the cylinder axis (30).

16. Implant according to any one of the preceding claims, **characterized in that** the spacing between the artificial articular surface (14) and the cylindrically shaped area (24) of the anchorage surface (22) is at its greatest in a plane of symmetry (36) of the implant (10) extending transversely to the cylinder axis (30).

17. Implant according to any one of the preceding claims, **characterized in that** the cylindrically shaped area (24) of the anchorage surface (22) extends at maximum over an angle range (25) of 180°, in particular over an angle range of 120° at maximum.

18. Implant according to claim 17, **characterized in that** the angle range (25) comprises about 110°.

19. Implant according to any one of the preceding claims, **characterized in that** the implant is a unicondylar implant (10) for replacement of a condyle surface (13) of a femur (12).

20. Implant set comprising a plurality of implants according to any one of the preceding claims, wherein radii of curvature of the artificial articular surfaces (14) vary by a spacing of 5 mm, in particular by 3 mm, preferably by 1 mm.

21. Implant set according to claim 20, **characterized in that** the implant set comprises implants (10', 10") configured symmetrically to one another.

## Revendications

1. Implant (10) destiné à se substituer à au moins une partie d'une surface articulaire naturelle d'un os (12) formant une partie d'une articulation, l'implant présentant une surface d'ancrage (22), qui s'applique contre l'os (12) résiduel, et une surface articulaire artificielle (14) pointant dans une direction s'éloignant de l'os (12), la surface d'ancrage (22) comprenant au moins une zone (24) de forme cylindrique, qui définit un axe de cylindre (30), l'implant étant d'une configuration telle que la surface articulaire artificielle (14) soit d'une courbure convexe en s'éloignant de l'axe de cylindre (30), et que la surface articulaire artificielle (14) présente une forme sphérique, l'axe de cylindre (30) contenant le centre (20) de la surface articulaire artificielle (14) de forme sphérique.

2. Implant selon la revendication 1, **caractérisé en ce que** la totalité de la surface d'ancrage (22) est de forme cylindrique.

3. Implant selon la revendication 1, **caractérisé en ce que** la surface d'ancrage (22) comprend une zone plane (26), et **en ce que** la zone plane (26) se raccorde à la zone (24) de forme cylindrique.

4. Implant selon la revendication 3, **caractérisé en ce que** dans la zone de transition (34) entre la zone plane (26) et la zone (24) de forme cylindrique, la zone plane (26) forme un plan tangent à la zone (24) de forme cylindrique.

5. Implant selon l'une des revendications 3 ou 4, **caractérisé en ce que** l'implant (10) présente un secteur d'ancrage plan (52) et un secteur d'ancrage courbe (54), **en ce que** le secteur d'ancrage plan (52) comprend la zone plane (26), et **en ce que** le secteur d'ancrage courbe (54) comprend la zone (24) de forme cylindrique.

6. Implant selon la revendication 1, **caractérisé en ce que** le secteur d'ancrage plan (52) présente un plan de symétrie (36), qui s'étend transversalement par rapport à l'axe de cylindre (30).

7. Implant selon l'une des revendications 6 ou 7, **caractérisé en ce que** le secteur d'ancrage courbe (54) présente un plan de symétrie (36), qui s'étend transversalement par rapport à l'axe de cylindre.

8. Implant selon l'une des revendications précédentes, **caractérisé en ce que** l'implant (10) présente un plan de symétrie (36), qui s'étend transversalement par rapport à l'axe de cylindre.

9. Implant selon l'une des revendications précédentes, **caractérisé en ce que** l'implant (10) présente des surfaces latérales (46a, 46b, 47a, 47b), qui relient mutuellement la surface articulaire artificielle (14) et la surface d'ancrage (22).

10. Implant selon la revendication 9, **caractérisé en ce que** les surfaces latérales (46a, 46b, 47a, 47b) présentent, parallèlement à la direction de l'axe de cylindre (30), une distance d'espacement réciproque (B) constante.

11. Implant selon l'une des revendications 9 ou 10, **caractérisé en ce que** les surfaces latérales (46a, 47a) sont courbes au moins par tronçons, de sorte qu'une distance des surfaces latérales (46a, 47a) à un plan s'étendant transversalement à l'axe de cylindre (30), varie.

12. Implant selon la revendication 11, **caractérisé en ce que** la distance des surfaces latérales (46a, 47a) au plan s'étendant transversalement à l'axe de cylindre (30), augmente à partir du secteur d'ancrage plan (52).

13. Implant selon l'une des revendications 11 ou 12, **caractérisé en ce que** les surfaces latérales (46a, 47a) du secteur d'ancrage courbe (54) sont courbes par rapport au plan (36) s'étendant transversalement à l'axe de cylindre (30).

14. Implant selon la revendication 12, **caractérisé en ce qu'**une courbure des surfaces latérales (46a, 47a) est constante.

15. Implant selon l'une des revendications précédentes, **caractérisé en ce que** la distance entre la surface articulaire artificielle (14) et la zone (24) de forme cylindrique de la surface d'ancrage (22), est constante dans un plan s'étendant transversalement à l'axe de cylindre (30).

16. Implant selon l'une des revendications précédentes, **caractérisé en ce que** la distance entre la surface articulaire artificielle (14) et la zone (24) de forme cylindrique de la surface d'ancrage (22), est la plus grande dans un plan de symétrie (36) de l'implant (10) s'étendant transversalement à l'axe de cylindre (30).

17. Implant selon l'une des revendications précédentes, **caractérisé en ce que** la zone (24) de forme cylindrique de la surface d'ancrage (22) s'étend au maximum sur une zone angulaire (25) de 180°, en particulier sur une zone angulaire d'au maximum 120°.

18. Implant selon la revendication 17, **caractérisé en ce que** la zone angulaire (25) couvre environ 110°.

19. Implant selon l'une des revendications précédentes, **caractérisé en ce que** l'implant est un implant unicondylaire (10) destiné à se substituer à une surface de condyle (13) d'un os de fémur (12).

20. Jeu d'implants comprenant une pluralité d'implants selon l'une des revendications précédentes, dans lequel des rayons de courbure des surfaces articulaires artificielles (14) varient selon un pas de 5 mm, notamment de 3 mm, de préférence de 1 mm.

21. Jeu d'implants selon la revendication 20, **caractérisé en ce que** le jeu d'implants comprend des implants (10', 10") de configuration mutuellement symétrique.
